(19) Europäisches Patentamt

**European Patent Office**

**Office européen des brevets**

(11) Veröffentlichungsnummer: **0 351 518 B1**

(12) EUROPÄISCHE PATENTSCHRIFT

(45) Veröffentlichungstag der Patentschrift: **09.12.92**

(21) Anmeldenummer: **89109318.9**

(22) Anmeldetag: **24.05.89**

(51) Int. Cl.⁵: **A61L 2/10**

(54) **Vorrichtung zur Bestrahlung von Medien mittels UV-Licht.**

(30) Priorität: **20.07.88 DE 3824647**

(43) Veröffentlichungstag der Anmeldung:
**24.01.90 Patentblatt 90/04**

(45) Bekanntmachung des Hinweises auf die
Patenterteilung:
**09.12.92 Patentblatt 92/50**

(84) Benannte Vertragsstaaten:
**AT BE CH DE ES FR GB IT LI NL SE**

(56) Entgegenhaltungen:
**DE-A- 2 119 961       DE-A- 2 400 430**
**FR-A- 2 579 079       US-A- 3 686 940**
**US-A- 4 309 616       US-A- 4 766 321**

(73) Patentinhaber: **WEDECO GESELLSCHAFT FÜR
ENTKEIMUNGSANLAGEN MBH
Daimlerstrasse 5
W-4900 Herford(DE)**

(72) Erfinder: **Wedekamp, Horst
Elverdisser Strasse 92
W-4900 Herford(DE)**

(74) Vertreter: **Thömen, Uwe, Dipl.-Ing.
Patentanwalt U. Thömen Zeppelinstrasse 5
W-3000 Hannover 1(DE)**

EP 0 351 518 B1

## Beschreibung

Die Erfindung betrifft eine Vorrichtung zur Bestrahlung von Medien mittels UV-Licht, beispielsweise für die Entkeimung von Flüssigkeiten und/oder Gasen durch UV-Licht, in der die UV-Lichtquellen nach dem Prinzip der sogenannten positiven Bestrahlungsgeometrie kranzförmig achsparallel um den mediumdurchströmten Rohrkörper angeordnet sind.

Eine Vorrichtung dieses Prinzips ist durch das Patent DE 21 19 961 bekannt.

Die Erzielung einer möglichst hohen Wirksamkeit mit geringstem Kostenaufwand erfordert eine Maximierung der Bestrahlungsstärken in der Bestrahlungskammer. Um hohe Bestrahlungsstärken an jedem Ort der durch den Rohrkörper gebildeten Bestrahlungskammer zu erzielen, ist es durch die genannte DE-PS 21 19 961 bekannt, das zu entkeimende Medium durch den für UV-Licht durchlässigen Rohrkörper aus Quarz zu führen und außen um den Rohrkörper herum eine Mehrzahl von achsparallel angeordneten UV-Lichtquellen vorzusehen. Jeder Lichtquelle ist dabei ein Reflektor zugeordnet, um eine möglichst große Strahlungsenergie für die Bestrahlung des in dem Rohrkörper fließenden Mediums zur Verfügung zu stellen.

Dise Art der Anordnung der Lichtquellen zum Rohrkörper zeichnet sich dadurch aus, daß durch Fokussierung die Strahlungsintensität mit zunehmendem Abstand zur Strahlenquelle zunimmt. In der Fachwelt ist diese Art der Bestrahlung unter dem Begriff "Positive Bestrahlungsgeometrie" bekannt geworden.

Diese bekannte Vorrichtung hat sich in der Praxis bewährt, nachteilig ist allerdings der niedrige Energietransfer in den Rohrkörper von nur etwa 55 %. Ein Großteil der von den UV-Lichtquellen emittierten Strahlung geht hier durch Rückreflektion in die Lichtquellen und durch Absorbtion verloren.

Der Erfindung liegt die Aufgabe zugrunde, durch optimierten Energietransfer und Leistungssteigerung der UV-Lichtquellen eine wesentliche Erhöhung der Bestrahlungsstärke, d.h., der Strahlendichte im zu behandelnden Fluid zu erzielen.

Dies erreicht die Erfindung bei der im Oberbegriff des Patentanspruchs 1 vorausgesetzten Vorrichtung durch die im kennzeichnenden Teil angegebenen Merkmale.

Mit der Erfindung ist es in vorteilhafter Weise möglich, Flachstrahler bei einer Vorrichtung gemäß DE-21 19 961 zur Bestrahlung von Flüssigkeiten und/oder Gasen einzusetzen.

Der Flachstrahler besitzt aufgrund seines flachovalen Querschnitts mit jeweils zwei Schmalseiten und zwei Breitseiten eine ausgeprägte Strahlungscharakteristik:
Das Maximum der Strahlung wird über die Breitseiten, lediglich ca. 30 % über die Schmalseiten des Strahlers emittiert.

Erfindungsgemäß wird ein Satz Flachstrahler in der Weise achsparallel um den Rohrkörper angeordnet, daß die Schmalseiten des Flachstrahlers jeweils in Richtung der Mittelachse des Rohrkörpers orientiert sind. Hierdurch erfolgt die Maximalemission der Breitseiten des Flachstrahlers tangential zum Rohrkörper. Mittels Spezialreflektoren werden sowohl dieser tangentiale als auch der dem Rohrkörper abgewandte Strahlungsfluß in den Rohrkörper umgelenkt.

Durch diese Anordnung der Flachstrahler wird der Wirkungsgrad des Energietransfers in den Rohrkörper gegenüber der bekannten Verwendung von Rundstrahlern gemäß der DE-PS 21 19 961 wesentlich erhöht.

Mit speziell geformten Reflektoren, z.B. parabolisch oder elliptisch, kann der Anteil der in den Strahler zurückreflektierten ultravioletten Strahlung auf praktisch 0 % reduziert werden. Unter Berücksichtigung der gerichteten Strahlungsverteilung und der Winkelabhängigkeit von Emission und Reflektion wird in der erfindungsgemäßen Anordnung von Flachstrahlern und Reflektoren eine Einbringung von 80 % - 90 % der Gesamtemission in das zu behandelnde Fluid erzielt.

Die erfindungsgemäße Anordnung von Flachstrahlern und Reflektoren beinhaltet auch die Kontaktkühlung der UV-Strahler sowohl direkt über den fluidführenden Rohrkörper als auch indirekt über die Reflektorschenkel. Hierfür liegen die UV-Strahler mit ihren Schmalseiten einerseits am Rohrkörper und andererseits an den Reflektorschenkeln an. Die Reflektoren führen ihrerseits die Wärme durch Kontakt zum Rohrkörper ab.

Vorteilhafte Ausgestaltungen und zweckmäßige Weiterbildungen der Erfindung ergeben sich aus den Unteransprüchen und der nachfolgenden Beschreibung.

Anhand der in der Zeichnung dargestellten Ausführungsbeispiele wird die Erfindung zum besseren Verständnis näher erläutert. Es zeigen:

Fig. 1     eine Vorrichtung mit drei um einen Rohrkörper angeordneten Flachstrahlern mit jeweils einem Reflektor,

Fig. 2     eine Darstellung gemäß Fig. 1, jedoch mit einem anderen Reflektor, und

Fig. 3     eine Darstellung gemäß Fig. 1, mit einem Reflektor mit gerade verlaufenden Reflektorschenkeln.

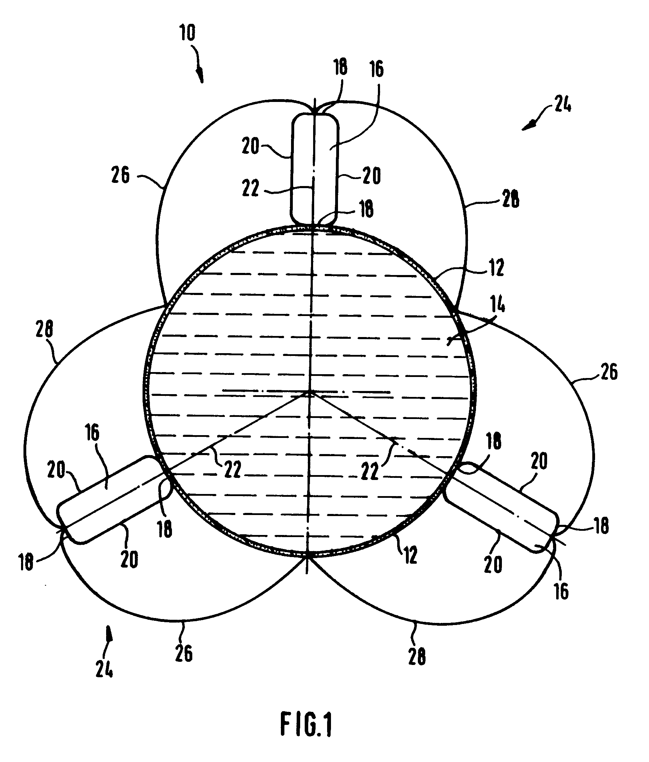

Die in Fig. 1 zu erkennende und als Ganzes mit der Bezugsziffer 10 bezeichnete Vorrichtung umfaßt einen Rohrkörper 12, der sich senkrecht zur Zeichenebene erstreckt und von einer zu entkeimenden Flüssigkeit 14 durchströmt ist. Der Mantel des Rohrkörpers 12 besteht aus Quarz und ist somit für UV-Strahlung durchlässig.

Außen um den Rohrkörper 12 herum sind im gleichen Abstand voneinander drei Flachstrahler 16 angeordnet. Jeder Flachstrahler 16 besitzt zwei Schmalseiten 18 und zwei Breitseiten 20. Ferner ist jedem Flachstrahler 16 ein Reflektor 24 mit jeweils zwei Reflektorschenkeln 26 und 28 zugeordnet.

Die achsparallel angeordneten Flachstrahler 16 sind so ausgerichtet, daß sich jeweils die durch die Schmalseiten 18 verlaufende Mittelachse 22 radial zum Rohrkörper 12 erstreckt.

Die Schmalseiten 18 liegen an dem Rohrkörper 12 an, und durch den so geschaffenen Kontakt zwischen Flachstrahler 16 und Rohrkörper 12 ergibt sich eine Kontaktkühlung.

Die Reflektorschenkel 26, 28 befinden sich mit ihren ersten Enden ebenfalls in Berührung mit dem Rohrkörper 12, und die zweiten Enden liegen an den äußeren Schmalseiten 18 der Flachstrahler 16 an. Auch durch die Reflektorschenkel 26 und 28 ergibt sich somit eine Kontaktkühlung.

Die Hauptstrahlungsrichtung der Flachstrahler 16 verläuft senkrecht zur Mittelachse 22 der Schmalseiten 18, d.h., der weitaus größte Teil tritt aus den Breitseiten 20 tangential zum Rohrkörper 12 aus. Mit Hilfe der Reflektorschenkel 26 und 28 wird sowohl der tangentiale als auch der dem Rohrkörper 12 abgewandte Strahlungsfluß der äußeren Schmalseite in den Rohrkörper 12 umgelenkt.

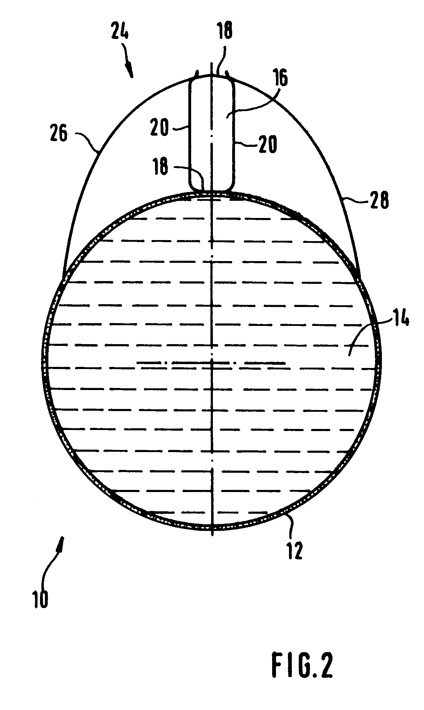

Während die Reflektorschenkel 26, 28 in Fig. 1 einen etwa elliptischen Verlauf aufweisen, zeigt Fig. 2 eine Ausführungsform, bei der die Reflektoren 24 parabolisch verlaufen (zur besseren Übersicht der Zeichnung ist in Fig. 2 der Einfachheit halber lediglich ein Flachstrahler 16 mit einem Reflektor 24 dargestellt). Die äußere Schmalseite des Flachstrahlers 16 wird hier nicht vom Reflektor 24 umfaßt.

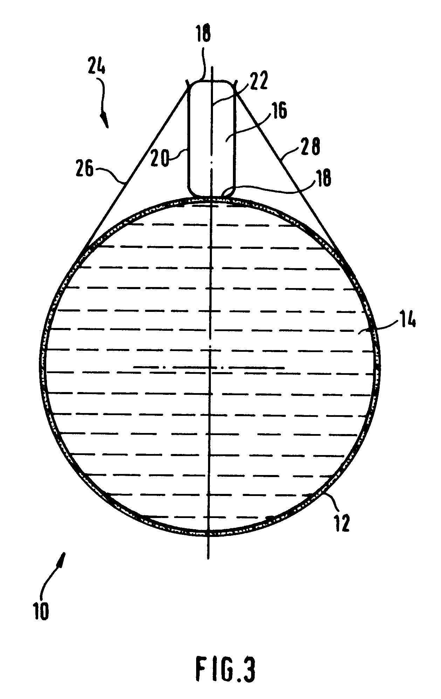

Bei der weiteren Ausführungsform gemäß Fig. 3 sind die Reflektorschenkel 26 und 28 eben. Wie in Fig. 2 ist eine Kontaktkühlung für den Flachstrahler 16 und durch die Reflektorschenkel 26, 28 vorgesehen.

Versuche haben ergeben, daß mit der erfindungsgemäßen Vorrichtung eine Verbesserung des Wirkungsgrades auf über 80 % gegenüber dem Wirkungsgrad von maximal 55 % der bekannten Vorrichtung gemäß der DE-PS 21 19 961 erreicht wird.

## Patentansprüche

1.  Vorrichtung zur Bestrahlung von Medien mittels UV-Licht, bestehend aus einem vom Medium durchströmten Rohrkörper (12) aus UV-durchlässigem Werkstoff, und mindestens zwei Stück außen achsparallel angeordneten UV-Lichtquellen mit Reflektoren, **dadurch gekennzeichnet,** daß die Lichtquellen durch UV-Flachstrahler (16) gebildet sind, die einen länglich, flachovalen Querschnitt mit Breit- und Schmalseiten (18, 20) aufweisen, und daß die Schmalseiten (18) der UV-Lichtquellen jeweils mit ihrer Mittelachse (22) auf den Mittelpunkt des Rohrkörperquerschnittes gerichtet sind.

2.  Vorrichtung nach Anspruch 1, dadurch gekennzeichnet, daß die Flachstrahler (16) mit ihren dem Rohrkörper (12) zugewandten Schmalseiten (18) am Rohrkörper (12) anliegen.

3.  Vorrichtung nach einem der vorhergehenden Ansprüche, dadurch gekennzeichnet, daß die Reflektoren (24) durch jeweils zwei Reflektorschenkel (26, 28) gebildet sind, und daß die Reflektorschenkel (26, 28) an ihren dem Rohrkörper (12) abgewandten Enden in Berührung mit dem Flachstrahler (16) sind.

4.  Vorrichtung nach Anspruch 3, dadurch gekennzeichnet, daß die Reflektorschenkel (26, 28) mit ihren dem Rohrkörper (12) zugewandten Enden am Rohrkörper (12) anliegen.

5.  Vorrichtung nach einem der vorhergehenden Ansprüche, dadurch gekennzeichnet, daß die Reflektoren (24) parabolisch ausgebildet sind.

6.  Vorrichtung nach einem der vorhergehenden Ansprüche 1 - 4, dadurch gekennzeichnet, daß die Reflektoren (24) in Form eines Kegelschnittes ausgebildet sind.

7.  Vorrichtung nach einem der vorhergehenden Ansprüche 1 - 4, dadurch gekennzeichnet, daß die Reflektoren (24) eben ausgebildet sind.

## Claims

1.  Device for irradiating fluids with UV light, consisting of a tube (12) of UV-transmitting material through which the fluid flows, and of at least two UV light sources with reflectors, arranged externally parallel to the axis, characterized in that the light sources are formed of UV flat-section radiators (16), which have an elongate, flatly oval cross-section with wide and narrow sides (18, 20), and that the narrow sides (18) of the UV light sources are each orientated with their central axis (22) towards the centre-point of the cross-section of the tube.

2.  Device according to Claim 1, characterized in that the flatsection radiators (16) bear with their narrow sides (18) which are towards the tube (12) on this tube (12).

3. Device according to one of the preceding Claims, characterized in that the reflectors (24) are each formed of two reflector arms (26, 28), and that the reflector arms (26, 28) are in contact with the flat-section radiator (16) at their ends remote from the tube (12).

4. Device according to Claim 3, characterized in that the reflector arms (26, 28) bear with their ends which are towards the tube (12) on this tube (12).

5. Device according to one of the preceding Claims, characterized in that the reflectors (24) are of parabolic shape.

6. Device according to one of the preceding Claims 1 to 4, characterized in that the reflectors (24) are shaped as a portion of a cone.

7. Device according to one of the preceding Claims 1 to 4, characterized in that the reflectors (24) are of flat construction.

**Revendications**

1. Dispositif pour l'irradiation de milieux à la lumière ultraviolette, comprenant un corps tubulaire traversé par le milieu en une matière perméable aux ultraviolets, et au moins deux sources extérieures de lumière ultraviolette avec réflecteurs, parallèles à l'axe du corps tubulaire, **caractérisé en ce que** les sources lumineuses sont des radiateurs plats ultraviolets (16) présentant une section oblongue en forme d'ovale plat avec des côtés larges et étroits (18, 20) et en ce que les axes médians (22) des côtés étroits (18) des sources de lumière ultraviolettes sont respectivement tournés vers le milieu de la section du corps tubulaire.

2. Dispositif suivant la revendication 1, **caractérisé en ce que** les radiateurs plats (16) avec leurs côtés étroits tournés vers le corps tubulaire (12), sont placés contre le corps tubulaire.

3. Dispositif suivant une des revendications précédentes, **caractérisé en ce que** les réflecteurs (24) sont respectivement formés par deux branches (26, 28) dont les extrémités distantes du corps tubulaire sont en contact avec le radiateur plat (16).

4. Dispositif suivant la revendication 3, **caractéri-**

**sé en ce que** les branches de réflecteur (26, 28) avec leurs extrémités tournées vers le corps tubulaire (12) sont en contact avec lui.

5. Dispositif suivant une des revendications précédentes, **caractérisé en ce que** les réflecteurs (24) sont de forme parabolique.

6. Dispositif suivant une des revendications précédentes 1 à 4, **caractérisé en ce que** les réflecteurs (24) ont section de forme conique.

7. Dispositif suivant une des revendications précédentes 1 à 4, **caractérisé en ce que** les réflecteurs (24) sont plans.

FIG.1

FIG.2

FIG.3